# EUROPEAN PATENT SPECIFICATION

(11) **EP 3 838 249 B1**
(45) Date of publication and mention of the grant of the patent: **07.01.2026**
(21) Application number: 19849980.8
(22) Date of filing: 31.07.2019
(51) Int. Cl.: A61K 8/04, A61K 8/891, A61Q 1/10, A61Q 19/00, A61K 8/06, A61Q 1/02, A61Q 1/04, A61Q 17/04

(54) **OIL-IN-WATER EMULSION COSMETIC COMPOSITION CONTAINING A CARBOXYLIC ACID MODIFIED SILICONE**
KOSMETISCHE ZUSAMMENSETZUNG AUS ÖL-IN-WASSER-EMULSION, DIE EIN MIT CARBONSÄURE MODIFIZIERTES SILIKON ENTHALTEN
COMPOSITION COSMÉTIQUE EN ÉMULSION HUILE DANS L'EAU CONTENANT UNE SILICONE MODIFIÉE PAR UN ACIDE CARBOXYLIQUE

(30) Priority: 15.08.2018 JP 2018152811
(43) Date of publication of application: 23.06.2021
(73) Proprietor: Dow Toray Co., Ltd., Tokyo 140-8617 (JP)
(72) Inventor: KONDO Junya, Tokyo 143-0015 (JP); HARAMIZU Satoshi, Tokyo 143-0015 (JP); PHAN Son Thanh, Ichihara-shi, Chiba 299-0108 (JP); MIYANO Jun, Tokyo 140-8617 (JP); KANZAKI Yasue, Ichihara-shi, Chiba 299-0108 (JP); HORI Seiji, Ichihara-shi, Chiba 299-0108 (JP)
(74) Representative: Murgitroyd & Company
(86) International application number: PCT/JP2019/029930
(87) International publication number: WO 2020/036061

(56) References cited:
- EP-A1- 3 132 789
- JP-A- 2002 146 189
- JP-A- 2015 203 026
- JP-A- H0 844 260
- US-A- 5 948 391

## Description

### [TECHNICAL FIELD]

The present invention relates to an oil-in-water emulsion cosmetic composition including a continuous aqueous phase and a discontinuous oil phase.

### [BACKGROUND ART]

Oil-in-water emulsion cosmetic compositions containing an aqueous phase as a continuous phase are widely used as make-up cosmetic compositions such as base cosmetic compositions such as emulsions, foundation cosmetic compositions, sunscreen agents, foundations, and other makeup cosmetic compositions such as eye shadow.

On the other hand, such cosmetic compositions typically have problems that the cosmetic film obtained by applying the cosmetic compositions has poor water resistance.

Thus, for example, as described in Japanese Unexamined Patent Application Publication No. 2004-91423, it has been proposed to blend, in a cosmetic composition containing an aqueous phase, a hydrophobic powder that has undergone hydrophobization treatment in order to impart water resistance to a cosmetic film.

However, because the surface of such hydrophobic powder is non-hydrophilic, it is difficult to disperse the hydrophobic powder in the aqueous phase, and there is a problem that the hydrophobic powder aggregates and settles out of phase over time.

Therefore, Japanese Unexamined Patent Application Publication No. 2015-203026 discloses the use of a prescribed carboxylic acid modified silicone under alkaline conditions in order to favorably disperse the hydrophobic powder in the aqueous phase and stabilize the cosmetic composition.

### [PRIOR ART DOCUMENTS]

### [PATENT DOCUMENTS]

Patent Document 1: Japanese Unexamined Patent Application Publication No. 2004-91423
Patent Document 2: Japanese Unexamined Patent Application Publication No. 2015-203026

### [SUMMARY OF THE INVENTION]

### [PROBLEMS TO BE SOLVED BY THE INVENTION]

The use of a prescribed carboxylic acid modified silicone can increase the stability of an oil-in-water emulsion cosmetic composition, in particular, an oil-in-water emulsion cosmetic composition containing a hydrophobic powder.

On the other hand, there is a demand for providing an oil-in-water emulsion cosmetic composition with high stability irrespective of presence or absence of a hydrophobic powder and further enhancing water resistance of a cosmetic film obtained by using the cosmetic composition.

An object of the present invention is to provide an oil-in-water emulsion cosmetic composition that has high stability and can provide a cosmetic film having excellent water resistance.

### [MEANS FOR SOLVING THE PROBLEMS]

The object of the present invention can be achieved by

An oil-in-water emulsion cosmetic composition containing:
(A) a carboxylic acid modified silicone that is liquid at room temperature (25°C) expressed by a structural formula (1): 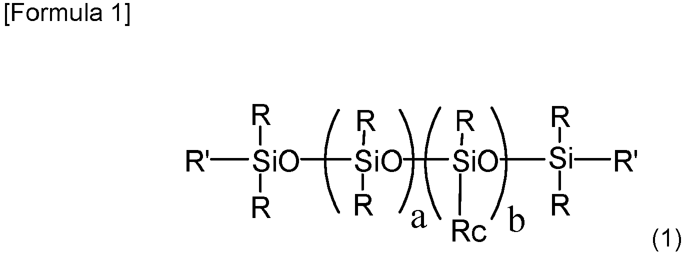 wherein:
   Rc represents a carboxyl group-containing organic group represented as a general formula: -R¹-(OR²)p-(O)w-R³-COOH , R¹ represents a linear or branched alkylene group having 2 to 22 carbon atoms, R² is a linear or branched alkylene group having 2 to 4 carbon atoms, R³ represents a bond (-) or a linear or branched alkylene group having 1 to 22 carbon atoms, p represents a number of 0 and w represents a number of 0**,**
   R represents a same or different alkyl or alkoxy group, having 1 to 22 carbon atoms, or phenyl group,
   R' is Rc or R,
   a and b each are a positive number, a + b is a number in a range of 2 to 20, and a/b is in a range of 0.3 to 3.0;
   an oil agent (B); and
   water (C).

In the structural formula (1), a/b is preferably from 0.3 to 2.0.

The cosmetic composition of the present invention preferably contains the carboxylic acid modified silicone (A) in a range of 0.1 to 15 mass%.

Note that "mass%" of the present specification is synonymous with "weight%", and is based on the total mass (total weight) of the cosmetic composition of the present invention, unless otherwise specified.

The oil agent (B) preferably contains at least one ultraviolet light absorbing agent.

The cosmetic composition of the present invention preferably contains the oil agent (B) in a range of 1 to 60 mass%.

The cosmetic composition of the present invention preferably contains water (C) in a range of 20 to 98 mass%.

The cosmetic composition of the present invention preferably further contains a basic compound (D).

The cosmetic composition of the present invention preferably contains the basic compound (D) in a range of 0.01 to 2.5 mass%.

The cosmetic composition of the present invention preferably further contains a polyhydric alcohol (E).

The cosmetic composition of the present invention preferably further contains one or more (F) selected from a film-forming agent (F1) and a water-soluble thickening agent (F2).

The cosmetic composition of the present invention preferably further contains a hydrophobic powder (G).

In the cosmetic composition of the present invention, the hydrophobic powder (G) can be dispersed in an oil phase and/or an aqueous phase.

The cosmetic composition of the present invention can contain a less than 5 mass% of surfactant other than the carboxylic acid modified silicone (A).

### [EFFECTS OF THE INVENTION]

The cosmetic composition of the present invention has high stability. Here, "stable" means uniform without causing phase separation over time.

In particular, the cosmetic composition of the present invention is stable with or without a hydrophobic powder.

Additionally, the cosmetic composition of the present invention can provide a cosmetic film having excellent water resistance.

Further, the cosmetic composition of the present invention can give a refreshing feeling when used.

The cosmetic film formed using the cosmetic composition of the present invention can be easily removed by using normal soap and water.

### [MODE FOR CARRYING OUT THE INVENTION]

As a result of diligent research, the present inventors discovered that an oil-in-water emulsion cosmetic composition that has high stability and can form a cosmetic film with excellent water resistance can be provided by using a carboxylic acid modified silicone that has a specific chemical structure and is in liquid form at room temperature (25°C), and thus completed the present invention.

The cosmetic composition of the present invention is stable even when the oil agent contains an ultraviolet light absorbing agent.

The cosmetic composition of the present invention is stable even when the cosmetic composition contains a film-forming agent and/or a water-soluble thickening agent.

When the cosmetic composition of the present invention contains a hydrophobic powder, water resistance of the obtained cosmetic film can be further increased.

When the cosmetic composition of the present invention contains the hydrophobic powder and a basic compound, the cosmetic composition of the present invention has high stability and can form a cosmetic film having more excellent water resistance.

When the cosmetic composition of the present invention does not contain a surfactant other than the carboxylic acid modified silicone (A), water resistance of the cosmetic film obtained by the cosmetic composition of the present invention can be further increased.

Examples of the surfactant other than the carboxylic acid modified silicone (A) that is essential to the cosmetic composition of the present invention are not particularly limited, but include anionic, cationic, nonionic, and amphoteric surfactants.

Note that in the present invention, "not contain" means substantially free of, and "substantially" means that the component can be contained at less than 5 mass% of the total mass of the cosmetic composition of the present invention. However, it is advantageous that the content is small, and the content is preferably 3 mass% or less, more preferably 2 mass% or less, and still more preferably 1 mass% or less. The cosmetic composition of the present invention is most preferably free of any surfactant other than the carboxylic acid modified silicone (A).

The cosmetic composition of the present invention will be described below in more detail.

### [Carboxylic acid modified silicone]

The cosmetic composition of the present invention is at least one carboxylic acid modified silicone (A) that is liquid at room temperature (25°C) and has a specific chemical structure. Note that the carboxylic acid modified silicone (A) is preferably liquid at room temperature (25°C) and at 1 atmosphere.

The carboxylic acid modified silicone (A) is expressed by the following structural formula (1): 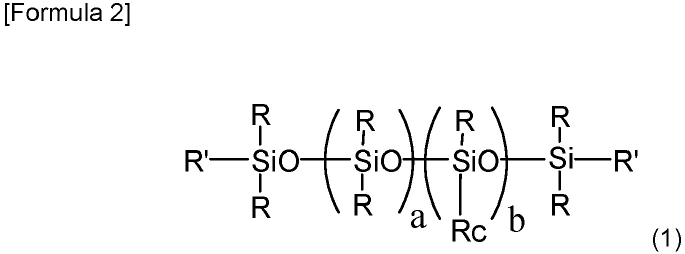 wherein:
Rc represents a carboxyl group-containing organic group represented as a general formula: -R¹-(OR²)p-(O)w-R³-COOH**,** R¹ represents a linear or branched alkylene group having 2 to 22 carbon atoms, R² is a linear or branched alkylene group having 2 to 4 carbon atoms, R³ represents a bond (-) or a linear or branched alkylene group having 1 to 22 carbon atoms, p represents a number of 0, and w represents a number of 0,
R represents a same or different alkyl or alkoxy group, having 1 to 22 carbon atoms, or phenyl group,
R' is Rc or R,
a and b each are a positive number, a ≥ 2 is preferable, and b ≥ 2 is preferable, a + b is a number in a range of 2 to 20, preferably 2 to 15, more preferably 2 to 10, and
a/b is in a range of 0.3 to 3.0, preferably from 0.3 to 2.5, more preferably from 0.3 to 2.0, and still more preferably from 0.5 to 2.0.

In the general formula representing the carboxyl group-containing organic group, R' is a linear or branched alkylene group having 2 to 22 carbon atoms, preferably a linear alkylene group having 2 to 12 carbon atoms, particularly preferably a linear alkylene group having 2 to 10 carbon atoms, and examples thereof include ethylene, propylene, trimethylene, butylene, pentamethylene, hexamethylene, heptamethylene, octamethylene, nonamethylene, decamethylene, undecamethylene, dodecamethylene, tridecamethylene, tetradecamethylene, pentadecamethylene, and hexadecamethylene groups.

Examples of the linear or branched alkylene group having 2 to 4 carbon atoms of R² include ethylene, propylene, trimethylene and butylene groups, and the ethylene group is particularly preferable.

Examples of the linear or branched alkylene group having 1 to 22 carbon atoms of R³ include methylene, ethylene, ethylethylene, propylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, and hexadecamethylene groups. Among the same, it is preferable that groups having the carbon atoms 1 to 12, particularly, the sum of carbon atoms of R' and R³, are from 2 to 22.

p represents a number of 0. In addition, w represents a number of 0. When both p and w are 0, the carboxyl group-containing organic group is expressed by the structure formula-(CₙH₂ₙ)-COOH, and preferably has a structure in which one carboxyl group is bonded to a silicon atom via a linear or branched alkylene group having 3 to 44 carbon atoms. In the formula, n is a number of 3 to 44, preferably a number of 3 to 20, more preferably a number of 3 to 16.

The carboxylic acid modified silicone (A) is not particularly limited as long as at least one carboxyl group-containing organic group is introduced into a side chain or an end of organosiloxane. Preferably, the carboxyl group-containing organic group is introduced into the side chain of the organosiloxane.

Therefore, in the carboxylic acid modified silicone (A) expressed by structural formula (1), for example, the silicone main chain is grafted with the carboxyl group-containing organic group as the side chain, the carboxyl group-containing organic group is added to one end of the silicone main chain, the carboxyl group-containing organic group is added to both ends of the silicone main chain, or the carboxyl group-containing organic group is added to both ends of the silicone main chain and the carboxyl group-containing organic group is grafted as the side chain, and optionally, a long chain alkyl group having 6 or more carbon atoms is further included. Most preferably, the silicone main chain is grafted with the carboxyl group-containing organic group as the side chain. Note that, when the carboxylic acid modified silicone has a long chain alkyl group, compounding stability with an organic oil agent such as hydrocarbon oil or organic cosmetic raw materials (in particular, ultraviolet light absorbing agent) may be improved.

The carboxylic acid modified silicone (A) expressed by structural formula (1) is preferably a carboxylic acid modified silicone in which R' is R and a carboxyl group-containing organic group expressed by the general formula:-R¹-(OR²) p-(O)w-R³-COOH is grafted to the silicone side chain, more preferably in which the silicone side chain has a plurality of the carboxyl group-containing organic groups, and still more preferably in which R' is R and the silicone side chain has a plurality of the carboxyl group-containing organic groups and a/b = 1.

In the structural formula (1), R is preferably a methyl group, an alkoxy group, or a phenyl group, but from the perspective of compounding stability with an organic oil agent such as hydrocarbon oil or organic cosmetic raw materials (in particular, a ultraviolet light absorbing agent), R may partially have a long chain alkyl group having 6 to 22 carbon atoms.

Such carboxylic acid modified silicone can be produced by any known method, for example, a method in which a dimethylpolysiloxane having a Si-H group and an unsaturated carboxylic acid ester compound are subjected to addition reaction under a platinum catalyst, and further saponified into carboxylic acid; a method in which a dimethylpolysiloxane having a Si-H group and an unsaturated carboxylic acid silyl ester or an allyloxycarboxylic acid silyl ester are subjected to addition reaction under a platinum catalyst and, after the reaction, are hydrolyzed to obtain a desired object; or a method of equilibrating reaction using bis(hydroxycarbonylethyl)tetramethyldisiloxane, cyclic siloxane and an acidic catalyst to obtain a carboxylic acid modified silicone at both ends (Silicone Handbook, edited by Kunio Ito, Nikkan Kogyo Shimbun, pp. 166-167). Furthermore, a product marketed as the trade name ES-5800 Formulation Aid manufactured by Dow Toray Co., Ltd.) is exemplified as the suitable carboxylic acid modified silicone of the present invention.

The cosmetic composition of the present invention preferably contains the carboxylic acid modified silicone (A) in a range of 0.1 to 15 mass% of the total mass of the cosmetic composition, more preferably in a range of 0.5 to 10 mass%, and still more preferably in a range of 1.0 to 5 mass%.

### [Oil agent]

The cosmetic composition of the present invention includes at least one oil agent (B). The oil agent forms an oil phase in the cosmetic composition of the present invention.

The "oil agent" of the present invention is generally used as a component of a cosmetic composition, and is not particularly limited. The oil agent is normally liquid at room temperature, but may be a solid such as a wax, or may be a highly viscous gum or paste shape described below.

The oil agent is preferably at least one liquid at 5 to 100°C selected from the group consisting of silicone oils, nonpolar organic compounds, and low polarity organic compounds.

Silicone oils are hydrophobic, and their molecular structure may be cyclic, linear, or branched. The viscosities of silicone oils at 25°C are usually in the range of 0.65 to 100,000 mm²/s, preferably in the range of 0.65 to 10,000 mm²/s.

Silicone oils include, for example, linear organopolysiloxanes, cyclic organopolysiloxanes, and branched organopolysiloxanes. Among these, linear organopolysiloxanes, cyclic organopolysiloxanes, and branched organopolysiloxanes that are volatile are preferable.

More specifically, examples of linear organopolysiloxanes include dimethylpolysiloxane capped at both molecular chain ends with trimethylsiloxy groups (dimethylsilicone having low viscosity such as 2 mPa·s or 6 mPa·s to high viscosity of 1,000,000 mPa·s), organohydrogenpolysiloxane, methylphenylpolysiloxane capped at both molecular chain ends with trimethylsiloxy groups, dimethylsiloxane/methylphenylsiloxane copolymer capped at both molecular chain ends with trimethylsiloxy groups, diphenylpolysiloxane capped at both molecular chain ends with trimethylsiloxy groups, dimethylsiloxane/diphenylsiloxane copolymer capped at both molecular chain ends with trimethylsiloxy groups, trimethylpentaphenyl trisiloxane, phenyl (trimethylsiloxy) siloxane, methyl alkyl polysiloxane capped at both molecular chain ends with trimethylsiloxy groups, dimethylpolysiloxane/methylalkylsiloxane copolymer capped at both molecular chain ends with trimethylsiloxy groups, dimethylsiloxane/methyl (3,3,3-trifluoropropyl) siloxane capped at both molecular chain ends with trimethylsiloxy groups, α,ω-dihydroxypolydimethylsiloxane, α,ω-diethoxypolymethylsiloxane, 1,1,1,3,5,5,5-heptamethyl-3-octyltrisiloxane, 1,1,1,3,5,5,5-heptamethyl-3-dodecyltrisiloxane, 1,1,1,3,5,5,5-heptamethyl-3-hexadecyltrisiloxane, tristrimethylsiloxymethylsilane, tristrimethylsiloxyalkylsilane, tetrakistrimethylsiloxysilane, tetramethyl-1,3-dihydroxydisiloxane, octamethyl-1,7-dihydroxytetrasiloxane, hexamethyl-1,5-diethoxytrisiloxane, hexamethyldisiloxane, octamethyltrisiloxane, higher alkoxy modified silicones, higher fatty acid modified silicones and dimethiconol.

Examples of cyclic organopolysiloxanes include hexamethylcyclotrisiloxane (D3), octamethylcyclotetrasiloxane (D4), decamethylcyclopentasiloxane (D5), dodecamethylcyclotetrasiloxane (D6), 1,1-diethylhexamethylcyclotetrasiloxane, phenylheptamethylcyclotetrasiloxane, 1,1-diphenylhexamethylcyclotetrasiloxane, 1,3,5,7-tetravinyltetramethylcyclotetrasiloxane, 1,3,5,7-tetramethylcyclotetrasiloxane, 1,3,5,7-tetracyclohexyltetramethylcyclotetrasiloxane, tris(3,3,3-trifluoropropyl) trimethylcyclotrisiloxane, 1,3,5,7-tetra(3-methacryloxypropyl)tetramethylcyclotetrasiloxane, 1,3,5,7-tetra(3-acryloxypropyl)tetramethylcyclotetrasiloxane, 1,3,5,7-tetra(3-carboxypropyl)tetramethylcyclotetrasiloxane, 1,3,5,7-tetra(3-vinyloxypropyl)tetramethylcyclotetrasiloxane, 1,3,5,7-tetra(p-vinylphenyl)tetramethylcyclotetrasiloxane, 1,3,5,7-tetra[3-(p-vinylphenyl)propyl]tetramethylcyclotetrasiloxane, 1,3,5,7-tetra(N-acryloyl-N-methyl-3-aminopropyl)tetramethylcyclotetrasiloxane and 1,3,5,7-tetra(N,N-bis(lauroyl)-3-aminopropyl)tetramethylcyclotetrasiloxane.

Examples of the branched organopolysiloxane include methyltristrimethylsiloxysilane, ethyltristrimethylsiloxysilane, propyltristrimethylsiloxysilane, tetrakistrimethylsiloxysilane, and phenyltristrimethylsiloxysilane.

As the nonpolar organic compound and the low polarity organic compound, a hydrocarbon oil and a fatty acid ester oil are preferable. These are components which are widely used, in particular, as substrates for make-up cosmetics.

Examples of the hydrocarbon oil include liquid paraffin, light liquid isoparaffin, heavy liquid isoparaffin, petrolatum, n-paraffin, isoparaffin, isododecane, isohexadecane, polyisobutylene, hydrogenated polyisobutylene, polybutene, ozokerite, ceresin, microcrystalline wax, paraffin wax, polyethylene wax, polyethylene polypropylene wax, squalane, squalene, pristane and polyisoprene.

Examples of the fatty acid ester oil include hexyl decyl octanate, cetyl octanate, isopropyl myristate, isopropyl palmitate, butyl stearate, hexyl laurate, myristyl myristate, oleyl oleate, decyl oleate, and octyldodecyl myristate. Hexyldecyl dimethyloctanoate, cetyl lactate, myristyl lactate, diethyl phthalate, dibutyl phthalate, lanolin acetate, propylene glycol diioylate, glyceryl tri2-ethylhexanoate, trimethylpropane tri-2-ethylhexanoate, ditriethylhexanoate Methylol propane, (isostearic acid/sebacic acid) ditrimethylol propane, trimethylol propane trioctanoate, trimethylol propane triisostearate, diisopropyl adipate, diisobutyl adipate, 2-hexyldecyl adipate, di-2-heptylune adipate decyl, diisostearyl malate, hydrogenated castor oil monoisostearate, octyldodecyl isostearate, isopropyl isostearate, isosetyl isostearate, ethylene glycol di-2-ethylhexanoate, cetyl 2-ethylhexanoate, tetra-2-pentaerythritol ethylhexanoate, octyldodecyl gum ester, ethyl oleate, octyldodecyl oleate, neopentyl glycol dicaprate, triethyl citrate, 2-ethylhexyl succinate, dioctyl succinate, isosetyl stearate, diisopropyl sebacate, sebacic acid di-2-ethylhexyl, diethyl sebacate, dioctyl sebacate, dibutyl octyl sebacate, cetyl parimitinate, octyldodecyl palmitate, octyl palmitate, 2-ethylhexyl palmitate, 2-hexyldecyl palmitate, 2-heptyl palmitate undecyl, cholesteryl 12-hydroxystearyl acid, dipentaerythritol fatty acid ester, 2-hexyldecyl myristate, ethyl laurate, N-lauroyl-L-glutamic acid-2-octyldodecyl ester, N-lauroyl-L-glutamate di(cholesteryl/behenyl/octyldodecyl), N-lauroyl-L-glutamate (cholesteryl/octyldodecyl), N-lauroyl-L-glutamate (phytosteryl/behenyl/octyldodecyl), N-lauroyl-L-glutamate di(phytosteryl/octyldodecyl), N-lauroyl sarcosin isopropyl, diisostearyl malate, neopentyl glycol dioctanoate, isodecyl neopentate, isotridecyl neopentanoate, isostearyl neopentate, isononane Isononyl acid, isotridecyl isononanoate, octyl isononanoate, isotridecyl isononanoate, diethylpentanediol dineopenate, methylpentanediol dineopenate, octyldodecyl neodecanoate, 2-butyl-2-ethyl-1,3-propanediol dioctanoate, tetra pentaerythrityl octanate, hydrogenated rosin pentaerythricyl, pentaerythricyl triethylhexanoate, (hydroxystearic acid/stearic acid/logonic acid) dipentaerythrityl, polyglyceryl tetraisostearate, polyglyceryl nonaisostearate-10, deca (erucic acid/Isostearic acid/ricinoleic acid) polyglyceryl-8, (hexyldecanoic acid/sebacic acid) diglyceryl oligoester, glycol distearate (ethylene glycol distearate), diisopropy dimer dilinoleate , diisostearyl dimer dilinoleate, phytosteryl isostearyl dimer dilinoleate, behenyl/phytosteryl dimer dilinoleate, phytosteryl/isostearyl/cetyl/stearyl/behenyl dimer dilinoleate, dimer dilinoleyl dimer dilinoleate, dimer dilinoleyl diisostearate, dimer dilinoleyl hydrogenated rosinate, hydrogenated castor oil dimer dilinoleate, hydroxyalkyl dimer dilinoleyl ether, glyceryl triisooctanoate, glyceryl triisostearate, glyceryl trimyristate, glyceryl triisopalmitate, trioctanoic acid glyceryl, glyceryl trioleate, glyceryl diisostearate, caprylic/capric triglyceride, tri (caprylic acid/capric acid/myristic acid/stearic acid) glyceryl, hydrogenated rosin triglyceride (hydrogenated ester gum), rosin triglyceride (Ester gum), glyceryl beecosanate, glyceryl di-2-heptyl undecanoate, diglyceryl myristate isostearate, co-acetate cholesteryl, cholesteryl nonanoate, cholesteryl stearate, cholesteryl isostearate, cholesteryl oleate, cholesteryl 12-hydroxystearate, macadamia nut oil fatty acid cholesteryl, macadamia nut oil fatty acid phytosteryl, phytosteryl isostearate, soft lanolin fatty acid cholesteryl, hard lanolin fatty acid cholesteryl, chain-branched fatty acid cholesteryl, long-chain α-hydroxy fatty acid cholesteryl, octyldodecyl ricinoleate, octyldodecyl lanolin fatty acid, octyldodecyl erucate, hardened isostearic acid, avocado oil fatty acid ethyl, and lanolin fatty acid isopropyl.

For example, higher alcohols having 10 to 30 carbon atoms can be used as the low polarity organic compound. When a higher alcohol is used as the emulsion stabilizing component, the amount of hydrophilic surfactant can be reduced, and the water resistance can be further improved. The higher alcohol is a saturated or unsaturated monohydric aliphatic alcohol, and the hydrocarbon group portion may be either linear or branched, but is more preferably linear. Examples of higher alcohols having 10 to 30 carbon atoms include lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, 2-decyltetradecinol, cholesterol, sitosterol, phytosterol, lanosterol, lanolin alcohol and hydrogenated lanolin alcohol. In the present invention, it is preferable to use a higher alcohol having a melting point of from 40 to 80°C alone, or to combine a plurality of higher alcohols so that the melting point is from 40 to 70°C.

The blending amount of the oil agent in the cosmetic composition of the present invention is not particularly limited, but is preferably 1 to 60 mass%, more preferably from 2 to 40 mass%, and still more preferably from 3 to 20 mass%, based on the total mass of the cosmetic composition.

### (Ultraviolet Light Absorbing Agent)

The oil agent (B) can contain at least one ultraviolet light absorbing agent. That is, the oil phase of the cosmetic composition of the present invention can contain the ultraviolet light absorbing agent. The ultraviolet light absorbing agent is preferably organic, more preferably lipophilic, and still more preferably oil soluble.

The oil-soluble ultraviolet light absorbing agent is not particularly limited as long as it is used in cosmetic compositions or skin external preparations, and examples thereof include those described below. The oil-soluble ultraviolet light absorbing agent can be used alone or as a mixture of two or more.

Cinnamic acid-based ultraviolet light absorbing agents such as benzyl paramethoxycinnamate, 2-ethylhexyl paramethoxycinnamate, and mono-2-ethylhexanoate glyceryl diparamethoxycinnamate;
Benzophenone-based ultraviolet light absorbing agents such as hydroxymethoxybenzophenone, dihydroxymethoxybenzophenone, dihydroxybenzophenone, and tetrahydroxybenzophenone;
Benzoic acid ester-based ultraviolet light absorbing agents such as paraaminobenzoic acid, ethyl paraaminobenzoate, glyceryl paraaminobenzoate, amyl paradimethylaminobenzoate, octyl paradimethylaminobenzoate, 4-[N, N-di (2-hydroxypropyl) amino] ethyl benzoate, and diethylaminohydroxy hexyl benzoyl benzoate;
Salicylic acid-based ultraviolet light absorbing agents such as ethylene glycol salicylate, phenyl salicylate, octyl salicylate, benzyl salicylate, paratershary butyl phenyl salicylate, homomentyl salicylate;
Triazine-based ultraviolet light absorbing agents such as ethylhexyltriazone (2,4,6-tris [4-(2-ethylhexyloxycarbonyl) anilino] 1,3,5-triazine), bisethylhexyloxyphenol methoxyphenyltriazine;
Other ultraviolet light absorbing agents such as 4-tarshary butyl-4'-methoxydibenzoylmethane, menthyl anthranilate, 2-(2-hydroxy-5-methylphenyl) benzotriazole, 2-ethylhexyl dimethoxybenzylidene dioxoimidazolidine propionate, octocrylene, dimethicodiethyl benzylmalonate.

Among these, the ultraviolet light absorbing agents such as 2-ethylhexyl paramethoxycinnamate, mono-2-ethylhexanate diparamethoxycinnamate, octyl salicylate, homomentyl salicylate, bisethylhexyloxyphenol methoxyphenyltriazine, dihydroxybenzophenone, octocrylene, 4-tashalibutyl-4'-methoxydibenzoylmethane, hexyl diethylaminohydroxybenzoyl benzoate can be selected to acquire a particularly high ultraviolet protection effect.

The blending amount of the ultraviolet light absorbing agent in the cosmetic composition of the present invention is not particularly limited, but is preferably from 0 to 30 mass%, more preferably from 0.1 to 20 mass%, based on the total mass of the cosmetic composition. However, in the cosmetic composition of the present invention, the ultraviolet light absorbing agent is an optional component, and may not be blended.

### [Water]

The cosmetic composition of the present invention contains water (C). The water forms an aqueous phase in the cosmetic composition of the present invention.

The blending amount of water in the cosmetic composition is not particularly limited, but is preferably 20 to 98 mass%, more preferably 30 to 95 mass%, and still more preferably from 40 to 90 mass%, based on the total mass of the cosmetic composition of the present invention.

### [Basic Compound]

The cosmetic composition of the present invention can include at least one basic compound (D).

The basic compound used in the present invention is not particularly limited as long as it is a compound that exhibits basicity when dissolved in water, and various types of inorganic compounds and organic compounds can be used. One or more basic compounds may be blended.

Examples of the organic compound include monoethanolamine, triethanolamine, 2-amino-2-methyl-1,3-propanediol, aminomethylpropanol, arginine, and guanidine.

Examples of the inorganic compound include sodium hydroxide, sodium carbonate, potassium hydroxide, potassium carbonate, calcium hydroxide, calcium carbonate, and ammonia, and among these, potassium hydroxide can be particularly preferably used.

The blending amount of the basic compound in the cosmetic composition of the present invention is not particularly limited, but in the case of a monovalent base per 1 mol of the carboxylic acid group contained in the blended carboxylic acid modified silicone, the carboxylic acid group/monovalent base (molar ratio) is preferably 1/0.5 to 1/1.5. Specifically, the blending amount of the base is preferably 0.01 to 2.5 mass%, more preferably 0.05 to 2.0 mass%, still more preferably 0.1 to 1.5 mass% based on the total mass of the cosmetic composition.

The pH of the cosmetic composition of the present invention may be acidic or alkaline, but is preferably weakly alkaline, and specifically, is preferably in the range of 7.1 to 9.5, and more preferably in the range of 7.2 to 8.5.

When the pH of the cosmetic composition of the present invention is alkaline, the carboxylic acid modified site of (A) the carboxylic acid modified silicone is anionized, thereby allowing the function of the carboxylic acid modified silicone (A) to suitably perform as a surfactant. Especially when the cosmetic composition of the present invention contains a hydrophobic powder, the hydrophobic powder can be suitably dispersed in the cosmetic composition of the present invention.

### [Polyhydric Alcohol]

The cosmetic composition of the present invention may contain at least one polyhydric alcohol (E).

The use of the polyhydric alcohol can adjust the feeling of moisturization and the sensation of use of the cosmetic composition of the present invention, and when the cosmetic composition of the present invention contains a hydrophobic powder, the hydrophobic powder can be dispersed in the aqueous phase well by mixing the component (A) and the hydrophobic powder with the polyhydric alcohol in advance, and then mixed with other components to prepare the cosmetic composition.

Examples of the polyhydric alcohol include sorbitol, xylitol, propylene glycol, dipropylene glycol, 1,3-butylene glycol, glycerin, diglycerin, and polyethylene glycol, and these polyhydric alcohols can be used independently or in combination of two or more. When the component (A) and the hydrophobic powder are mixed together with the polyhydric alcohol in advance, the liquid polyhydric alcohol can be uniformly dispersed in the aqueous phase, and among the same, propylene glycol, dipropylene glycol, 1,3-butylene glycol, glycerin, diglycerin, and combinations thereof are preferable.

The blending amount of the polyhydric alcohol in the cosmetic composition of the present invention is not particularly limited, but is preferably 0.3 to 30 mass%, more preferably 0.5 to 25 mass%, and still more preferably from 1 to 20 mass%, based on the total mass of the cosmetic composition of the present invention.

### [Film-Forming Agent and Water Soluble Thickening Agent]

The cosmetic composition of the present invention can contain one or more (F) selected from film-forming agents (F1) and water-soluble thickening agents (F2).

### [Film-forming Agent]

General-purpose film-forming agents for cosmetic compositions can be used as the film-forming thickening agent. For example, hydrophilic and/or hydrophobic organic polymers can be used as the film-forming agent.

For example, at least one selected from the group consisting of nonionic polymers, cationic polymers, an anion polymer, and amphoteric polymers described below can be used as the hydrophilic organic polymers.

Suitable nonionic polymers include, for example, nonionic polymeric compounds such as polyvinyl pyrrolidone (Rubiscol K, manufactured by BASF), vinyl pyrrolidone/vinyl acetate copolymer (Rubiscol VA manufactured by BASF), vinyl pyrrolidone/dimethylaminoethyl methacrylate copolymer (Copolymer 937 manufactured by ISP), vinyl caprolactam/vinyl pyrrolidone/dimethylaminoethyl methacrylate copolymer (Copolymer VC713 manufactured by ISP), polyvinyl alcohol, polyoxypropylene butyl ether and styrene/acrylate copolymer (Epitex-66 manufactured by DowChemical).

Suitable cationic polymers include, for example, cationic polymer compounds such as quaternary compounds of vinylpyrrolidone/dimethylaminoethylmethacrylate (GAFQUAT, manufactured by ISP), methylvinylimidazolidum chloride/vinylpyrrolidone copolymer (Rubicote, manufactured by BASF), cationized cellulose, cationized starch, cationized guar gum, vinylpyrrolidone/N, N-dimethylaminoethylmethacrylate copolymer diethylsulfate and diallyl quaternary ammonium salt polymers.

Suitable anionic polymers include, for example, cationic polymer compounds such as acrylate/methacrylate ester copolymers (Plascize manufactured by Goo Chemical Co., Ltd.), vinyl acetate/crotonic acid copolymer (Resin 28-1310, manufactured by NSC Corporation), vinyl acetate/crotonic acid/vinyl neodecanate copolymer (Resin 28-2930 manufactured by NSC Corporation), methylvinylether/maleic acid half ester (GANTREZ ES manufactured by ISP), tert-butyl acrylate/ethyl acrylate/methacrylic acid copolymer (Luvimer manufactured by BASF), vinyl pyrrolidone/vinyl acetate/vinyl propionate copolymer (Luviskol VAP manufactured by BASF), vinyl acetate/crotonic acid copolymer (Luviset CA manufactured by BASF), vinyl acetate/crotonic acid/vinyl pyrrolidone copolymer (Luviset CAP manufactured by BASF), vinyl pyrrolidone/acrylate copolymer (Luviflex manufactured by BASF), acrylate/acrylamide copolymer (Ultrahold manufactured by BASF), vinyl acetate/butyl maleate/isobornyl acrylate copolymer (Advantage manufactured by ISP), acrylic alkanolamine, and urethane-modified acrylic polymer represented by WO 2005/054341 and WO 2008/004502.

Suitable amphoteric polymers include, for example, amphoteric acetates of dialkylaminoethylmethacrylate polymers (Yucaformer manufactured by Mitsubishi Chemical Corporation), octylacrylamide acrylate/hydroxypropyl acrylate/butylaminoethyl methacrylate copolymer (AMPHOMER manufactured by NSC Corporation) and octyl acrylamide-butylaminoethyl methacrylate-hydroxypropyl methacrylate-acrylate copolymers.

On the other hand, the hydrophobic organic polymers are preferably oil soluble, and examples thereof include rosin acid based resins such as pentaerythritrit rosin acid and glyceryl rosin acid, candelilla resin, polyvinyl acetate resin and polyisobutylene. Note that the candelilla resin is a resin obtained by fractionating candelilla wax with an organic solvent, wherein the resin content is preferably 65 mass% or more, and more preferably 85 mass% or more.

The oil-soluble film-forming agent is more preferably a silicone agent;
and still more preferably at least one selected from a group consisting of a silicone resin containing M units and Q units,
silicone acrylate,
a silicone resin containing T units; and
silicone resin gum.

The silicone resin containing M units and Q units may be any silicone resin normally used in cosmetic compositions. The silicone resin containing M units and Q units (D1) may be any silicone resin as long as it includes triorganosiloxy units (M units) (for example, organo groups are only alkyl groups such as methyl groups or aryl groups such as alkyl groups such as methyl groups and allyl groups such as vinyl groups or aryl groups such as phenyl groups) and siloxy units (Q units). Examples thereof include MQ resins, MDQ resins, MDTQ resins (D represents diorganosiloxy units; for example, organo groups are only alkyl groups such as methyl groups or aryl groups such as alkyl groups such as methyl groups and allyl groups such as vinyl groups or aryl groups such as phenyl groups). More specifically, trimethyl siloxysilicate, polyalkyl siloxysilicate, trimethyl siloxysilicate containing dimethylsiloxy units, and alkyl (perfluoroalkyl) siloxysilicate are exemplified. These silicone resins are oil-soluble, and those that can be dissolved in tetracyclosiloxane (D4) or pentacyclosiloxane (D5) are particularly preferable.

Among these, trimethylsiloxysilicate is preferred. Examples of commercially available products include 749Fluid (manufactured by Dow Corning Toray Co., Ltd.), X-21-5595, KF-7312J, and KF-7312F (both available from Shin-Etsu Chemical Co., Ltd.). These may be used independently, or may be used in combination as appropriate.

Examples of the silicone acrylate include copolymer having a polyalkyl acrylate skeleton and a dimethicone polymer grafted to an alkyl ester side chain, such as a cyclopentasiloxane (and) acrylate/dimethicone copolymer (KP-545 manufactured by Shin-Etsu Chemical Co., Ltd.) and a methyltrimethicone (and) acrylate/dimethicone copolymer (KP-549 manufactured by Shin-Etsu Chemical Co., Ltd.). Also, a copolymer having a polyalkyl acrylate skeleton and a siloxane dendron structure grafted to an alkyl ester side chain, such as FA4001CM, FA4002ID, FA4103, FA4003DM, FA4004ID (manufactured by Dow Corning Toray Co., Ltd.), which is a (acrylates/polytrimethylsiloxy methacrylate) copolymer. These may be used independently, or may be used in combination as appropriate.

The silicone resin containing T units may be any silicone resin normally used in cosmetic compositions. The silicone resin containing T units (D3) may be any silicone resin as long as it has monoorganosiloxane units (T unit) (e.g., organo groups are alkyl groups such as methyl groups, or allyl groups such as vinyl groups, or aryl groups such as phenyl groups), and examples thereof include MTQ resins, MDTQ resins, TD resins, TQ resins and TDQ resins. It is particularly preferable that these silicone resins are oil-soluble and can be dissolved in D4 and D5.

Specific examples of the silicone resin containing T units include 670Fluid, which is polypropylsilsesquioxane, and SW-8005 C30 resin wax (manufactured by Dow Corning Toray Co., Ltd.), which is alkyl(C30-45)dimethylsilyl polypropylsilsesquioxane. These may be used independently, or may be used in combination as appropriate.

Examples of the silicone resin gum include FC-5002IDD (manufactured by Dow Corning Toray Co., Ltd.), which is a (trimethylsiloxysilicate/dimethiconol) crosspolymer.

### (Water-soluble Thickening Agent)

General-purpose thickening agents for cosmetic compositions can be used as the water-soluble thickening agent. The water-soluble thickening agent may be, for example, a hydrophilic organic polymer, and may have a function as a film-forming agent.

The use of the water-soluble thickening agent can adjust the viscosity and the sensation of use of the cosmetic composition of the present invention, and further improve the storage stability.

Examples of the water-soluble thickening agent include carboxyvinyl polymers, sodium polyacrylate, polyethylene glycol, acrylic acid alkyl methacrylate copolymers, polyoxyethylene polyoxypropylene block copolymer, polyvinyl alcohol, polyvinylpyrrolidone, polyvinyl methyl ether, methyl cellulose, ethyl cellulose, hydroxyethyl cellulose, hydroxypropyl cellulose, hydroxypropyl methylcellulose, cationized cellulose, sodium alginate, propylene glycol ester alginate, guar gum, locust bean gum, carrageenan, xanthan gum, dextran and bentonite, and is preferably a carboxyvinyl polymer, acrylic acid alkyl methacrylate copolymer, polyvinyl alcohol, polyvinylpyrrolidone, and hydroxypropyl methyl cellulose. These water-soluble thickening agents can be used independently or in combination of two or more.

The blending amount of the film-forming agent and/or water-soluble thickening agent in the cosmetic composition of the present invention is not particularly limited, but is preferably 0.01 to 5 mass%, more preferably from 0.05 to 2 mass%, and still more preferably from 0.1 to 2 mass%, based on the total mass of the cosmetic composition.

### [Hydrophobic Powder]

The cosmetic composition of the present invention may contain at least one hydrophobic powder (G).

The "powder" in the present invention is generally used as a component of a cosmetic composition, and includes white and colored pigments, microparticles (including so-called nanoparticles) such as ultraviolet scattering agents, and extender pigments. The white and colored pigments are used for coloring cosmetic compositions and the like, while the extender pigments are used for improving the feel of cosmetic compositions. As the "powder" of the present invention, white and colored pigments commonly used in cosmetic compositions, as well as extender pigments can be used without particular limitation. It is preferred to blend one or more powders.

The shape of the powder (spherical shape, rod shape, needle shape, plate shape, irregular shape and spindle shape), particle size (aerosol, fine particles and pigment grade), and the particle structure (porous and non-porous) are not limited, but the average primary particle size is preferably in the range of 1 nm to 100 µm.

Examples of the powder include inorganic powders, organic powders, surfactant metal salt powders (metal soaps), colored pigments, pearl pigments, metal powder pigments, and composites of these. Specifically, examples of the inorganic powders include titanium oxide, zirconium oxide, zinc oxide, cerium oxide, magnesium oxide, barium sulfate, calcium sulfate, magnesium sulfate, calcium sulfate, magnesium carbonate, talc, mica, kaolin, sericite, muscovite, synthetic mica, phlogopite, lepidolite, biotite, lithia mica, silicic acid, silicic anhydride, aluminum silicate, sodium silicate, sodium magnesium silicate, magnesium silicate, magnesium aluminum silicate, calcium silicate, barium silicate, strontium silicate, metal tungstate, hydroxyapatite, vermiculite, hydrargilite, bentonite, montmorillonite, hectorite, zeolite, ceramic powder, dicalcium phosphate, alumina, aluminum hydroxide, boron nitride, and boron nitride; examples of the organic powders include polyamide powder, polyester powder, polyethylene powder, and polypropylene powder, polystyrene powder, polyurethane powder, polystyrene powder, benzoganamine powder, polymethylbenzoganamine powder, polytetrafluoroethylene powder, polymethyl methacrylate powder, cellulose, silk powder, nylon powder, 12-nylon, 6-nylon, silicone powder, polymethylsilsesquioxane spherical powder, styrene/acrylic acid copolymer, divinylbenzene/styrene copolymer, vinyl resin, urea resins, phenol resin, fluororesin, silicon resin, acrylic resin, melamine resin, epoxy resin, polycarbonate resin, microcrystalline fiber powder, starch powder, and lauroyl lysine; examples of the surfactant metal salt powders include zinc stearate, aluminum stearate, calcium stearate, magnesium stearate, zinc myristate, magnesium myristate, zinc palmitate, zinc laurate, zinc cetyl phosphate, calcium cetyl phosphate, and sodium zinc cetyl phosphate; examples of the colored pigments include inorganic red pigments such as red oxide, iron oxide, iron hydroxide, iron titanate, inorganic brown pigments such as γ-iron oxide, inorganic yellow pigments such as yellow iron oxide, inorganic black pigments such as black iron oxide, carbon black, inorganic purple pigments such as manganese violet, cobalt violet, inorganic green pigments such as chromium hydroxide, chromium oxide, cobalt oxide, cobalt titanate, inorganic blue pigments such as prussian blue, ultramarine blue, tar dyes such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, Orange No. 207 and those obtained by laking the tar dyes, those obtained by laking natural dyes such as carmine acid, lacquemic acid, carthamine, braziline, and chrosine; examples of the pearl pigments include titanium oxide-coated mica, titanated mica, iron oxide-treated titanated mica, titanium oxide-coated mica, bismuth oxychloride, titanium oxide-coated bismuth oxychloride, titanium oxide-coated talc, fish scale foil and titanium oxide-coated colored mica; oxidized titanium oxide coated mica, oxychlorinated bismuth, titanium oxide coated bismuth oxychloride, titanium oxide coated tantalum foil, fish scaly foil, and titanium oxide coated colored mica; and examples of the metal powder pigments include metal powders such as aluminum, gold, silver, copper, platinum, and stainless steel.

When the surface of the powder itself is not hydrophobic, the surface is preferably subjected to hydrophobizing treatment. Note that these hydrophobic powders may be complexed together.

The hydrophobizing treatment is not particularly limited, and the powder is treated with various hydrophobizing surface treatment agents, for example, organosiloxane treatment such as methyl hydrogen polysiloxane (methicone in the Japanese cosmetics labeling name) treatment, (dimethicone/methicone) copolymer (hydrogen dimethicone in Japanese cosmetics labeling name) treatment, dimethylpolysiloxane (dimethicone in Japanese cosmetics labeling name) treatment, silicone resin treatment, silicone gum treatment, acrylic silicone treatment, and fluorinated silicone treatment; metal soap treatment such as zinc stearate treatment; silane treatment such as silane coupling agent treatment and alkylsilane treatment; fluorine compound treatment such as perfluoroalkylsilane, perfluoroalkyl phosphate ester salt, and perfluoro-ether treatment; amino acid treatment such as N-lauroyl-L-lysine treatment; oil treatment such as a squalane treatment; and acrylic treatments such as alkyl acrylate treatment. One or more of these treatments can be used in combination.

The powder may be subjected to hydrophobic surface treatment using the component (A) or other carboxylic acid modified silicone. For example, some or all of the powder may be surface-treated using a part of the component (A) and blended into the cosmetic composition of the present invention.

Among these treatments, treatment with the silicone compound is preferable in terms of water resistance and ease of dispersion by the carboxylic acid modified silicone, and the treatment with methylhydrogenpolysiloxane, (dimethicone/methicone) copolymer, dimethylpolysiloxane, and alkylsilane is particularly preferable. It should be noted that even when the surface is treated with the component (A) or other carboxylic acid modified silicone, there is a practical advantage in that the water resistance is excellent and the dispersion stability is excellent.

A silicone elastomer powder may also be used as the hydrophobic powder. The silicone elastomer powder is a crosslinked product of a liner diorganopolysiloxane mainly composed of a diorganosiloxy unit (D unit), and can be suitably obtaind by crosslinking an organohydrogenpolysiloxane having a silicon-bonded hydrogen atom at the side chain or end and a diorganopolysiloxane having an unsaturated hydrocarbon group such as an alkenyl group at the side chain or end under a hydrosilylation reaction catalyst. Since the silicone elastomer powder is soft, elastic, and excellent in oil absorption compared to the silicone resin powder composed of a T unit and a Q unit, the silicone elastomer powder can absorb oil and fat on the skin and prevent cosmetic collapse.

The silicone elastomer powder may have various shapes, such as spherical, flat, or indefinite. The silicone elastomer powder may be in the form of an oil dispersion. The cosmetic composition of the present invention is a silicone elastomer powder having a particle shape, a primary particle diameter thereof determined by observation using an electron microscope and/or an average primary particle diameter measured by laser diffraction/scattering method falls within a range of 0.1 to 50 µm, and a silicone elastomer powder having a spherical primary particle shape can be suitably compounded. The silicone elastomer constituting the silicone elastomer powder is preferably a silicone elastomer having a hardness of 80 or less, more preferably 65 or less according to JIS K 6253 "Hardness testing method for rubber, vulcanized or thermoplastic" as measured by type-A durometer.

The silicone elastomer powder may optionally be subjected to a surface treatment with a silicone resin or silica. Examples of the surface treatment include those described in JP 2-243612 A, JP 8-12545 A, JP 8-12546 A, JP 8-12524 A, JP 9-241511 A, JP 10-36219 A, JP 11-193331 A, and JP 2000-281523 A. The silicone elastomer powder corresponds to the crosslinked silicone powder listed in the "Japanese Cosmetic Ingredients Codex".

Commercial products of silicone elastomeric powders include, for example, Trefil E-506S, Trefil E-508, 9701 Cosmetic Powder, and 9702 Powder, available from Dow Corning Toray Co., Ltd. The silicone elastomeric powders may be surface-treated, and examples of the surface treatment agent include, for example, methylhydrogenpolysiloxane, silicone resin, metal soap, silane coupling agent, inorganic oxide such as silica and titanium oxide, fluorine compound such as perfluoroalkylsilane and perfluoroalkylphosphate ester salt.

Among these hydrophobic powders, hydrophobized fine particle inorganic powders are preferably used in terms of the ultraviolet light blocking effect, and among these hydrophobic powders, hydrophobic particulate titanium oxide and/or hydrophobized particulate zinc oxide are preferable. The particle size of the hydrophobized particulate titanium oxide and/or hydrophobized particulate zinc oxide is preferably 1 to 200 nm in terms of the ultraviolet light blocking effect and dispersibility, and more preferably 10 to 80 nm. Furthermore, in the present invention, hydrophobized inorganic pigment powder or pearl pigment powder may be used as hydrophobic inorganic particles, or a combination of the hydrophobized fine particle inorganic powder described above and the hydrophobized inorganic pigment powder may be used.

The hydrophobic powder (G) is dispersible in the oil phase and/or aqueous phase. When the basic compound (D) is used, the hydrophobic powder (G) can be well dispersed in the aqueous phase, and when the polyhydric alcohol (E) is used, the hydrophobic powder (G) can be more uniformly dispersed in the aqueous phase.

The blending amount of the hydrophobic powder in the cosmetic composition of the present invention is not particularly limited, but is preferably 1 to 40 mass%, more preferably 2 to 35 mass%, still more preferably 3 to 30 mass%, still more preferably 4 to 25 mass%, and still more preferably 5 to 20 mass%, based on the total mass of the cosmetic composition.

### [Optional Components]

Other components ordinarily used in cosmetic compositions can be added to the cosmetic composition of the present invention within a range that does not hinder the effect of the present invention, and examples of other such components include: hydrophilic powders, moisturizing agents other than the component (E), thickening agents other than the component (F2), antiseptic agents, antimicrobial agents, perfumes, salts, antioxidants, pH adjusting agents other than the component (D), chelating agents, refreshing agents, anti-inflammatory agents, physiologically active components (skin lightening agents, cell activating agents, rough skin improving agents, circulation promoters, skin astringents, anti-seborrheic agents, etc.), vitamins, amino acids, nucleic acids, hormones, and inclusion compounds. Other components are not particularly limited.

### [Manufacturing Method]

The manufacturing steps of the cosmetic composition of the present invention may be any steps and are not particularly limited as long as the steps can mix the above components to prepare the oil-in-water emulsion cosmetic composition containing the carboxylic acid modified silicone (A) that has the specific chemical structure describe above and is liquid at room temperature (25°C), the oil agent (B), and water (C).

If necessary, at least one selected from the group consisting of the above components (D), (E), (F), and (G) may be further mixed.

### [Usage Method]

The cosmetic composition of the present invention may be in the form of cream, gel, emulsion, or liquid. For example, the cosmetic composition of the present invention can be used as a base cosmetic composition such as milk, cream or serum; a sunscreen agent; and makeup cosmetic compositions such as foundations, eye shadows, eyeliners, and water powders, as well as sunscreen agents for hair and scalp, and temporary hair colorants.

The cosmetic composition of the present invention can also be used as a precursor for a cosmetic composition (premix or cosmetic raw material).

The cosmetic composition of the present invention is in the form of an oil-in-water emulsion composition, and because the water constituting the continuous phase is in direct contact with the skin, much more fresh sensation of use can be provided.

The cosmetic composition of the present invention is preferably a skin cosmetic composition, and is more preferably applied onto weak acid skin, and, for example, it is still more preferable to apply onto skin having a pH of 5.1 to 7.0.

In addition, when the cosmetic composition of the present invention is applied onto the skin at an applied amount of 0.5 mg/cm², after an elapse of 30 minutes, the pH of the application surface is preferably 7.0 or lower, and more preferably 6.7 or lower.

Because the cosmetic composition of the present invention is stable and can form a cosmetic film having excellent water resistance on the skin, it is difficult to cause makeup loss due to sweat and rain, and has excellent cosmetic durability.

### [Examples]

Hereinafter, the present invention is described in greater detail with reference to the following examples.

### (Synthesis Example 1)

230.67 g of trimethylsilyl undecylenate and 0.042 g of a platinum-1,3-divinyl-1,1,3,3-tetramethyldisiloxane complex were put into a flask equipped with a stirrer, a reflux condenser, a dropping funnel, and a thermometer, and 129.33 g of Si-Hsiloxane expressed by the following general formula was dropped thereinto so as to maintain the range of 70 to 80°C.

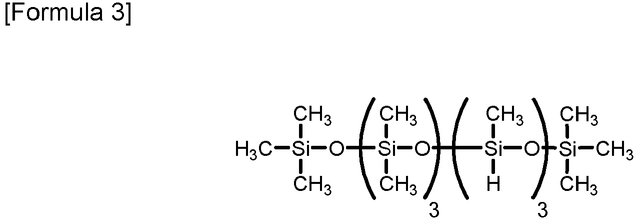

After the completion of dropping, the mixture was aged for 2 hours at 110°C, and then the loss of Si-H bonds was confirmed by the hydrogen generation method. A low boiling point content was distilled off under reduced pressure. Thereafter, 90 g of deionized water was added, and the mixture was aged under reflux for 4 hours for deprotection. Thereafter, the low boiling point content was again removed under reduced pressure to obtain a compound 1. As a result of analysis, it was confirmed that the chemical structure of the compound 1 was expressed by the following chemical formula:

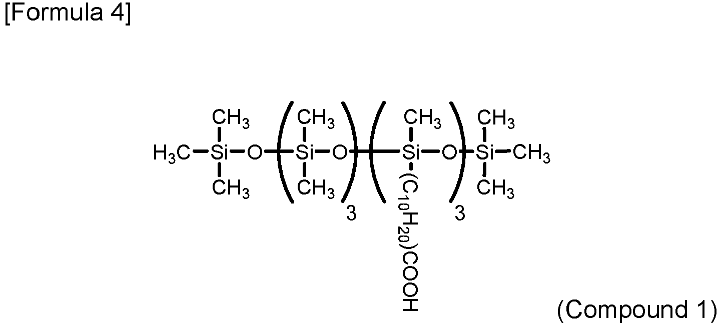

### (Synthesis Example 2)

283.39 g of trimethylsilyl undecylenate and 0.054 g of a platinum-1,3-divinyl-1,1,3,3-tetramethyldisiloxane complex were put into a flask equipped with a stirrer, a reflux condenser, a dropping funnel, and a thermometer, and 100.61 g of Si-Hsiloxane expressed by the following general formula was dropped thereinto so as to maintain the range of 70 to 80°C.

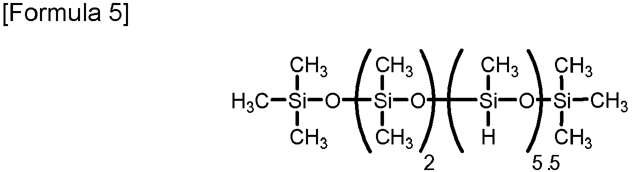

After the completion of dropping, the mixture was aged for 2 hours at 110°C, and then the loss of Si-H bonds was confirmed by the hydrogen generation method. A low boiling point content was distilled off under reduced pressure. Thereafter, 96 g of deionized water was added, and the mixture was aged under reflux for 4 hours for deprotection. Thereafter, the low boiling point content was again removed under reduced pressure to obtain a compound 2. As a result of analysis, it was confirmed that the chemical structure of the compound 2 was expressed by the following chemical formula:

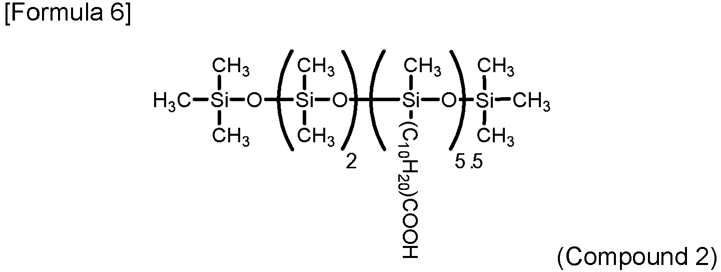

### (Synthesis Example 3)

159.00 g of trimethylsilyl undecylenate and 0.035 g of a platinum-1,3-divinyl-1,1,3,3-tetramethyldisiloxane complex were put into a flask equipped with a stirrer, a reflux condenser, a dropping funnel, and a thermometer, and 141.00 g of Si-Hsiloxane expressed by the following general formula was dropped thereinto so as to maintain the range of 70 to 80°C.

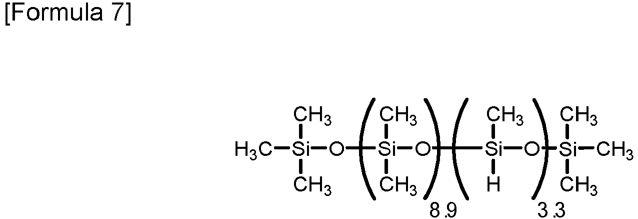

After the completion of dropping, the mixture was aged for 2 hours at 110°C, and then the loss of Si-H bonds was confirmed by the hydrogen generation method. A low boiling point content was distilled off under reduced pressure. Thereafter, 150 g of deionized water was added, aged at reflux for 4 hours, and deprotection was performed. Thereafter, the low boiling point content was again removed under reduced pressure to obtain a compound 3. As a result of analysis, it was confirmed that the chemical structure of the compound 3 was expressed by the following chemical formula:

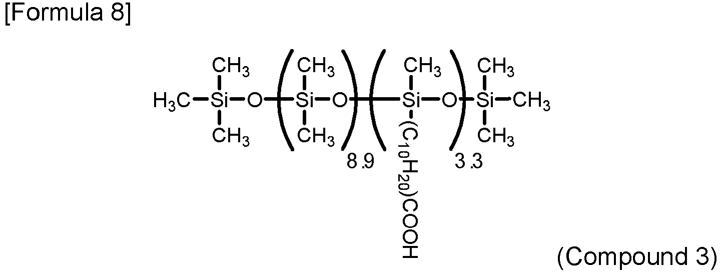

### (Synthesis Example 4)

292.40 g of trimethylsilyl undecylenate and 0.047 g of a platinum-1,3-divinyl-1,1,3,3-tetramethyldisiloxane complex were put into a flask equipped with a stirrer, a reflux condenser, a dropping funnel, and a thermometer, and 107.60 g of Si-Hsiloxane expressed by the following general formula was dropped thereinto so as to maintain the range of 70 to 80°C.

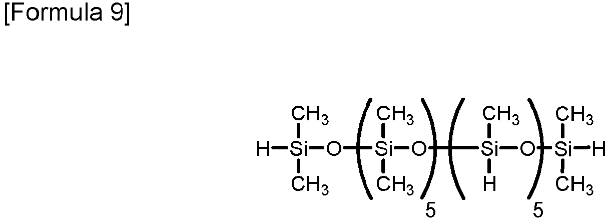

After the completion of dropping, the mixture was aged for 2 hours at 110°C, and then the loss of Si-H bonds was confirmed by the hydrogen generation method. A low boiling point content was distilled off under reduced pressure. Thereafter, 200 g of deionized water was added, and the mixture was aged under reflux for 4 hours for deprotection. Thereafter, the low boiling point content was again removed under reduced pressure to obtain a compound 4. As a result of analysis, it was confirmed that the chemical structure of the compound 4 was expressed by the following chemical formula:

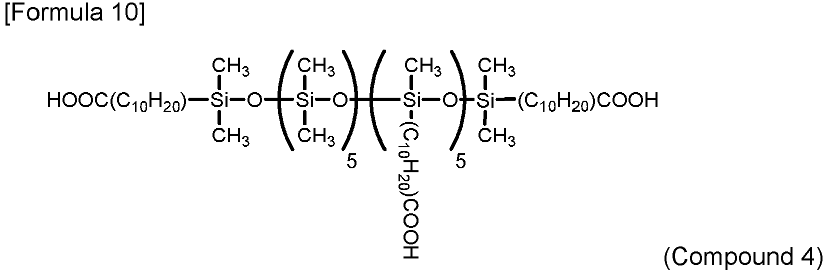

### (Synthesis Example 5)

51.44 g of trimethylsilyl undecylenate and 0.035 g of a platinum-1,3-divinyl-1,1,3,3-tetramethyldisiloxane complex were put into a flask equipped with a stirrer, a reflux condenser, a dropping funnel, and a thermometer, and 128.56 g of Si-Hsiloxane expressed by the following general formula was dropped thereinto so as to maintain the range of 70 to 80°C.

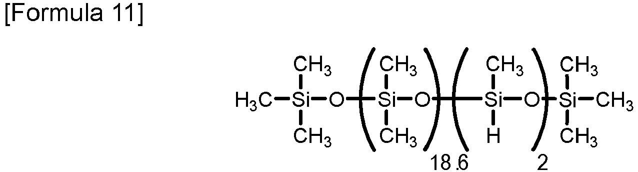

After the completion of dropping, the mixture was aged for 2 hours at 110°C, and then the loss of Si-H bonds was confirmed by the hydrogen generation method. A low boiling point content was distilled off under reduced pressure. Thereafter, 90 g of deionized water was added, and the mixture was aged under reflux for 4 hours for deprotection. Thereafter, the low boiling point content was again removed under reduced pressure to obtain a compound 5. As a result of analysis, it was confirmed that the chemical structure of the compound 5 was expressed by the following chemical formula:

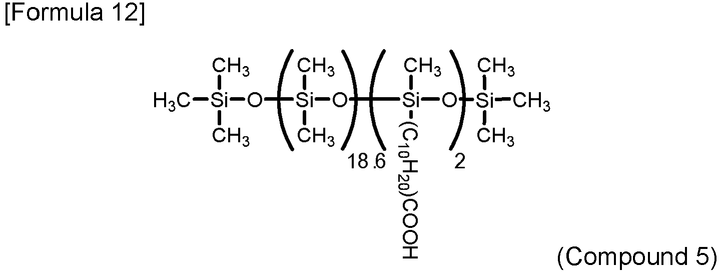

### (Synthesis Example 6)

632.55 g of trimethylsilyl undecylenate and 0.119 g of a platinum-1,3-divinyl-1,1,3,3-tetramethyldisiloxane complex were put into a flask equipped with a stirrer, a reflux condenser, a dropping funnel, and a thermometer, and 168.23 g of Si-Hsiloxane expressed by the following general formula was dropped thereinto so as to maintain the range of 70 to 80°C.

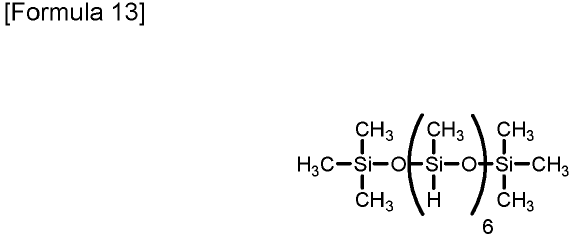

After the completion of dropping, the mixture was aged for 2 hours at 110°C, and then the loss of Si-H bonds was confirmed by the hydrogen generation method. A low boiling point content was distilled off under reduced pressure. Thereafter, 221.27 g of deionized water was added, and the mixture was aged under reflux for 4 hours for deprotection. Thereafter, the low boiling point content was again removed under reduced pressure to obtain a compound 6. As a result of analysis, it was confirmed that the chemical structure of the compound 6 was expressed by the following chemical formula:

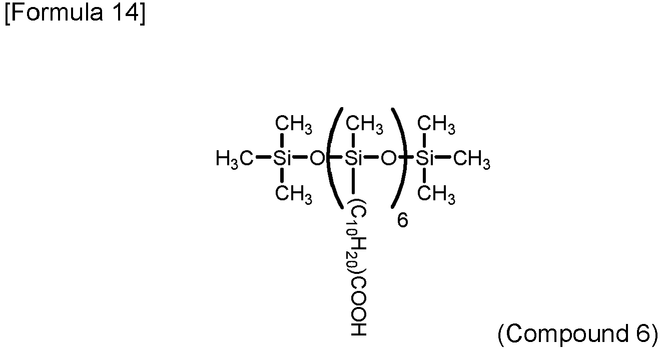

### (Synthesis Example 7)

100 g of 1,1,1,3,5,5,5-heptamethyltrisiloxane and 0.02 g of toluene solution of platinum-1,3-divinyl-1-1,3,3-tetramethyldisiloxane complex were put into a flask equipped with a stirrer, a reflux condenser, a dripping funnel, and a thermometer, and 105 g of trimethylsilyl undecylenate was dropped thereinto so as to maintain the range of 70 to 100°C. After the completion of dropping, the mixture was aged for 2 hours at 110°C, and then the loss of Si-H bonds was confirmed by the hydrogen generation method. A low boiling point content was distilled off under reduced pressure. Then, water was added, and the mixture was aged under reflux for 4 hours for deprotection. Thereafter, the low boiling point content was again removed under reduced pressure to obtain a compound 7. As a result of analysis, it was confirmed that the chemical structure of the compound 7 was expressed by the following chemical formula:

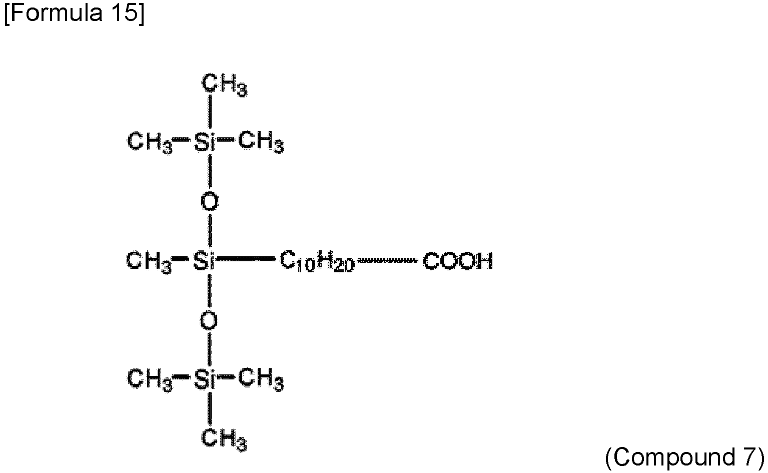

### [Examples 1 to 4 and Comparative Examples 1 and 2]

The compositions of Example 1 to 4 and Comparative Example 1 to 2 were manufactured by the manufacturing method described below using the components shown in Table 1. The blending amount of each component in Table 1 is represented in "mass%" ("weight%") unless otherwise specified.

### (Manufacturing Method)

(1) Components in a phase A are mixed.
(2) Components in a phase B are mixed.
(3) The mixture of the components in the phase B is added little by little to the mixture of the components in the phase A at normal room temperature to prepare a gel emulsion.
(4) Components in a phase C are mixed with the gel emulsion to prepare a cosmetic composition.

**[Table 1]**

| Phase | Component | Examples 1 | Examples 2 | Examples 3 | Examples 4 | Comparative Example 1 | Comparative Example 2 |
|---|---|---|---|---|---|---|---|
| | Water | 0.25 | 0.25 | 0.25 | 0.25 | 0.25 | 0.25 |
| | Potassium hydroxide | 0.25 | 0.25 | 0.28 | 0.17 | 0.24 | 0.08 |
| | Glycerin | 5 | 5 | 5 | 5 | 5 | 5 |
| | Compound 1 | 1.5 | - | - | - | - | - |
| A | Compound 2 | - | 1.5 | - | - | - | - |
| | Compound 3 | - | - | 1.5 | - | - | - |
| | Compound 4 | - | - | - | 1.5 | - | - |
| | Compound 5 | - | - | - | - | 1.5 | - |
| | PEG-12 dimethicone | - | - | - | - | - | 1.5 |
| B | Ethylhexyl methoxycinnamate | 3 | 3 | 3 | 3 | 3 | 3 |
| | Cyclopentasiloxane | 3 | 3 | 3 | 3 | 3 | 3 |
| | Caprylic/capric triglyceride | 4 | 4 | 4 | 4 | 4 | 4 |
| C | Water | 83 | 83 | 83 | 83 | 83 | 83 |
| Emulsion Stability | | ▪ | ▪ | ▪ | ▪ | Δ | Δ |

### (Evaluation of Emulsion Stability)

The compositions of Examples 1 to 4 and Comparative Examples 1 to 2 were stored in a constant temperature bath at 50°C for 30 days, and the presence or absence of separation of the oil phase and the aqueous phase was visually **observed,** and storage stability was evaluated according to the following criteria.
□: After storage for 30 days, separation of the oil phase and the aqueous phase does not occur.
Δ: After storage for 30 days, separation of the oil phase and the aqueous phase is observed.
×: Separation of the oil phase and the aqueous phase is observed immediately after preparation.

The results are shown in Table 1.

As is clear from Examples 1 to 4, a stable emulsion can be obtained when the carboxylic acid modified silicone (A) that is expressed by the above structural formula (1) and is liquid at room temperature (25°C) is used. On the contrary, as is clear from Comparative Example 1, when a carboxylic acid modified silicone that does not correspond to the carboxylic acid modified silicone (A) is used, a stable emulsion cannot be obtained. Furthermore, as is clear from Comparative Example 2, a stable emulsion cannot be obtained when the carboxylic acid modified silicone is not used.

### [Examples 5 and 6]

The compositions of Examples 5 and 6 were manufactured by the manufacturing method described below using the components shown in Table 2. The blending amount of each component in Table 2 is represented in "mass%" ("weight%") unless otherwise specified.

### (Manufacturing Method)

(1) Components in a phase A are mixed.
(2) Components in a phase B are mixed.
(3) The mixture of the components in the phase B is added little by little to the mixture of the components in the phase A at normal room temperature to prepare a gel emulsion.
(4) Components in a phase C are mixed with the gel emulsion to prepare a cosmetic composition.

**[Table 2]**

| Phase | Component | Examples 5 | Examples 6 |
|---|---|---|---|
| A | Water | 0.25 | 0.25 |
| | Potassium hydroxide | 0.25 | 0.25 |
| | Glycerin | 5 | 5 |
| | Compound 1 | 1.5 | 1.5 |
| B | Ethylhexyl methoxycinnamate | 6 | - |
| | Ethylhexyl methoxycinnamate (and) Diethylaminohydroxybenzoyl hexyl benzoate | - | 6 |
| | Caprylic/capric triglyceride | 4 | 4 |
| C | Water | 83 | 83 |
| Emulsion Stability | | □ | □ |

### (Evaluation of Emulsion Stability)

The compositions of Examples 5 and 6 were stored in a constant temperature bath at 50°C for 30 days, and the presence or absence of separation of the oil phase and the aqueous phase was visually observed, and storage stability was evaluated according to the following criteria.
□: After storage for 30 days, separation of the oil phase and the aqueous phase does not occur.
Δ: After storage for 30 days, separation of the oil phase and the aqueous phase is observed.
×: Separation of the oil phase and the aqueous phase is observed immediately after preparation.

The results are shown in Table 2.

As is clear from Table 2, the compositions of the present invention are stable, even with various ultraviolet light absorbing agents.

### [Examples 7 to 9]

The compositions of Examples 7 to 9 were manufactured by the manufacturing method described below using the components shown in Table 3. The blending amount of each component in Table 3 is represented in "mass%" ("weight%") unless otherwise specified.

### (Manufacturing Method)

(1) Components in a phase A are mixed.
(2) Components in a phase B are mixed.
(3) The mixture of the components in the phase B is added little by little to the mixture of the components in the phase A at normal room temperature to prepare a gel emulsion.
(4) Components in a phase C are mixed with the gel emulsion to prepare a cosmetic composition.

**[Table 3]**

| Phase | Component | Examples 7 | Examples 8 | Examples 9 |
|---|---|---|---|---|
| A | Water | 0.25 | 0.25 | 0.25 |
| | Potassium hydroxide | 0.25 | 0.25 | 0.25 |
| | Glycerin | 5 | 5 | 5 |
| | Compound 1 | 1.5 | 1.5 | 1.5 |
| B | Ethylhexyl methoxycinnamate | 3 | 3 | 3 |
| | Cyclopentasiloxane | 0.75 | - | 0.75 |
| | Cyclopentasiloxane (and) trimethylsiloxysilicate (polymer concentration 40 mass%) | 2.25 | - | - |
| | Cyclopentasiloxane (and) (acrylates/polytrimethylsiloxy methacrylate) copolymer (polymer concentration 30 mass%) | - | 3 | - |
| | Isododecane (and) (trimethylsiloxysilicate/dimethiconol ) crosspolymer (polymer concentration 40 mass%) | - | - | 2.25 |
| | Caprylic/capric triglyceride | 4 | 4 | 4 |
| C | Water | 83 | 83 | 83 |
| Emulsion Stability | | □ | □ | □ |

### (Evaluation of Emulsion Stability)

The compositions of Examples 7 to 9 were stored in a constant temperature bath at 50°C for 30 days, and the presence or absence of separation of the oil phase and the aqueous phase was visually observed, and storage stability was evaluated according to the following criteria.
□: After storage for 30 days, separation of the oil phase and the aqueous phase does not occur.
Δ: After storage for 30 days, separation of the oil phase and the aqueous phase is observed.
×: Separation of the oil phase and the aqueous phase is observed immediately after preparation.

The results are shown in Table 3.

As is clear from Table 3, the compositions of the present invention are stable, even with various film-forming agents.

### [Examples 10 to 11 and Comparative Examples 3 to 10]

The compositions of Example 10 to 11 and Comparative Example 3 to 10 were manufactured by the manufacturing method described below using the components shown in Table 4. The blending amount of each component in Table 4 is represented in "mass%" ("weight%") unless otherwise specified.

### (Manufacturing Method)

(1) Components in a phase A are mixed.
(2) Components in a phase B are mixed.
(3) The mixture of the components in the phase B is added little by little to the mixture of the components in the phase A at normal room temperature to prepare a gel emulsion.
(4) Components in a phase C are mixed with the gel emulsion to prepare a cosmetic composition.

**[Table 4]**

| Phase | Component | Examples 10 | Examples 11 | Comparative Example 3 | Comparative Example 4 | Comparative Example 5 |
|---|---|---|---|---|---|---|
| A | Water | 0.50 | 0.50 | 0.50 | 0.50 | 0.50 |
| | Potassium hydroxide | 0.53 | 0.50 | 0.50 | 0.61 | 0.45 |
| | Glycerin | 6.00 | 8.00 | 6.00 | 6.00 | 6.00 |
| | Compound 1 | 2.00 | 3.00 | - | - | - |
| | Isostearic acid | - | - | 2.00 | - | - |
| | Compound 6 | - | - | - | 2.00 | - |
| | Compound 7 | - | - | - | - | 2.00 |
| | Compound 5 | - | - | - | - | - |
| B | Cetyl ethylhexanoate | 5.00 | 13.00 | 5.00 | 5.00 | 5.00 |
| | Ethylhexyl methoxycinnamate | 7.50 | - | 7.50 | 7.50 | 7.50 |
| | Ethylhexyl salicylate | 5.00 | - | 5.00 | 5.00 | 5.00 |
| | Homomenthyl salicylate | 3.00 | - | 3.00 | 3.00 | 3.00 |
| | Triethoxycaprylylsilane treated titanium oxide | - | 8.00 | - | - | - |
| | Tocopherol | 0.05 | - | 0.05 | 0.05 | 0.05 |
| C | Water | 59.57 | 56.15 | 59.60 | 59.49 | 59.65 |
| | Dipropylene glycol | 9.00 | 9.00 | 9.00 | 9.00 | 9.00 |
| | Pentylene glycol | 1.00 | 1.00 | 1.00 | 1.00 | 1.00 |
| | Phenoxyethanol | 0.40 | 0.40 | 0.40 | 0.40 | 0.40 |
| | Xanthan gum | 0.05 | 0.05 | 0.05 | 0.05 | 0.05 |
| | (Acrylates /alkyl acrylate (C10-C30) Crosspolymer | 0.40 | 0.40 | 0.40 | 0.40 | 0.40 |
| | Total | 100. 00 | 100. 00 | 100. 00 | 100. 00 | 100. 00 |
| | Evaluation results | | | | | |
| | Stability | □ | □ | □ | □ | □ |
| | Water resistance | □ | □ | Δ | Δ | Δ |

**[Table 4 Continued]**

| Comparative Example 6 | Comparative Example 7 | Comparative Example 8 | Comparative Example 9 | Comparative Example 10 |
|---|---|---|---|---|
| 0.50 | 0.50 | 0.50 | 0.50 | 0.50 |
| 0.31 | 0.60 | 0.82 | 0.62 | 0.36 |
| 6.00 | 8.00 | 8.00 | 8.00 | 8.00 |
| - | - | - | - | - |
| - | 3.00 | - | - | - |
| - | - | 3.00 | - | - |
| - | - | - | 3.00 | - |
| 2.00 | - | - | - | 3.00 |
| 5.00 | - | - | - | - |
| 7.50 | 13.00 | 13.00 | 13.00 | 13.00 |
| 5.00 | - | - | - | - |
| 3.00 | - | - | - | - |
| - | 8.00 | 8.00 | 8.00 | 8.00 |
| 0.05 | - | - | - | - |
| 59.79 | 56.05 | 55.83 | 56.03 | 56.29 |
| 9.00 | 9.00 | 9.00 | 9.00 | 9.00 |
| 1.00 | 1.00 | 1.00 | 1.00 | 1.00 |
| 0.40 | 0.40 | 0.40 | 0.40 | 0.40 |
| 0.05 | 0.05 | 0.05 | 0.05 | 0.05 |
| 0.40 | 0.40 | 0.40 | 0.40 | 0.40 |
| 100. 00 | 100. 00 | 100. 00 | 100. 00 | 100. 00 |
| | | | | |
| Δ | □ | | □ | × |
| Δ | Δ | - | Δ | - |

### (Evaluation of Emulsion Stability)

The compositions of Examples and Comparative Examples were stored in a constant temperature bath at 50°C for 30 days, and the presence or absence of separation of the oil phase and the aqueous phase was visually observed, and storage stability was evaluated according to the following criteria.
□: After storage for 30 days, separation of the oil phase and the aqueous phase does not occur.
Δ: After storage for 30 days, separation of the oil phase and the aqueous phase is observed.
Í: Separation of the oil phase and the aqueous phase is observed immediately after preparation.

The results are shown in Table 4.

### (Evaluation of Water Resistance)

An evaluation target was applied uniformly onto the inside of the forearm of 20 female panelists with an application amount of 0.5 mg/cm², and after an elapse of 30 minutes, water droplets of water having a pH of 7.0 were gently dripped, and water droplets formed on the surface were photographed, and the contact angle was measured using a θ/2 method based on the shape and evaluated according to the criteria described below.

: Average value of contact angles is 80 degrees or more.
□: Average value of contact angles is 65 degrees or more and less than 80 degrees.
Δ: Average value of contact angles is 40 degrees or more and less than 65 degrees.
×: Average value of contact angles is less than 40 degrees.

The results are shown in Table 4.

As is clear from the results shown in Table 4, the compositions of Examples 10 and 11 in which a carboxylic acid modified silicone (A) that is represented by the above structural formula (1) and is liquid at room temperature (25°C) is used, exhibited excellent water resistance, and also have excellent stability even when the compositions contain a large amount of organic ultraviolet light absorbing agent or hydrophobic powder.

On the other hand, the compositions of Comparative Examples 3 and 7 in which a higher fatty acid (isostearic acid), which is an exemplary anionic surfactant, was neutralized with a base and used as a fatty acid soap, and the compositions of Comparative Examples 5 and 9 using the compound 7 that does not correspond to the carboxylic acid modified silicone (A) and has a/b of 0/1 in the structural formula (1) had inferior water resistance.

Furthermore, the composition of Comparative Examples 4 and 8 using the compound 6 that does not correspond to the carboxylic acid modified silicone (A) and has a/b of 0/6 in the structural formula (1) exhibited inferior water resistance, and stable emulsion could not be obtained when the composition contains a hydrophobic powder.

Furthermore, the composition of Comparative Examples 6 and 10 using the compound 5 having a/b of 18.6/2 had inferior stability.

Specific formulation examples of the composition of the present invention are shown below. The blending amount of each component in the table represents "mass%" ("weight%") unless otherwise specified. Note that, in the following formulation examples, in addition to the compound 1 described above, a carboxylic acid modified silicone marketed as the trade name ES-5800 Formulation Aid (manufactured by Dow Toray Co., Ltd.) can be suitably used as the carboxylic acid modified silicone (A).

### (O/W foundation)

**[Table 5]**

| Component | | Blending amount |
|---|---|---|
| Phase A | | |
| 1 | (A) Carboxylic acid modified silicone | 1.5 |
| 2 | Glycerin | 5 |
| 3 | 50% KOH aqueous solution | 0.5 |
| 4 | Caprylic/capric triglyceride | 4 |
| 5 | Cyclopentasiloxane | 3 |
| 6 | Ethylhexyl methoxycinnamate | 7.5 |

| Phase B | | |
|---|---|---|
| 7 | Acrylates copolymer (polymer concentration 30 mass%) | 3 |
| 8 | Water | Residual |
| 9 | Triethanolamine | Appropriate amount |
| 10 | Phenoxyethanol | 0.9 |

| Phase C | | |
|---|---|---|
| 11 | (A) Carboxylic acid modified silicone | 0.83 |
| 12 | Butylene glycol | 8.3 |
| 13 | Pigment mixture | 10 |
| 14 | Aminomethyl propanol | 0.13 |

| Phase D | | |
|---|---|---|
| 15 | (Divinyl dimethicone/dimethicone) copolymer (and) C12-13 pareth -3 (and) C12-13 pareth-23 (polymer concentration: 60 mass%) | 4.2 |

### (Manufacturing Method)

(1) Components 1 to 3 are mixed.
(2) Components 4 to 6 are mixed.
(3) While stirring the mixture obtained in (1) above, the mixture obtained in (2) above is gradually added thereto to form a phase A.
(4) Components in a phase B is mixed to form the phase B.
(5) Components in a phase C are mixed using three rolls to form the phase C.
(6) The phase A and the phase C are added to the phase B and mixed together.
(7) A phase D is added to the mixture of the phases A, B, and C phase, and the mixture is stirred to be uniform.

### (OIW foundation powder dispersion type in oil)

**[Table 6]**

| Component | | Blending amount |
|---|---|---|
| 1 | Water | 0.5 |
| 2 | Potassium hydroxide | 0.7 |
| 3 | Glycerin | 10 |
| 4 | (A) Carboxylic acid modified silicone | 5 |
| 5 | Diisostearyl malate | 2 |
| 6 | Triethoxycaprylylsilane treated iron oxide | 1.9 |
| 7 | Triethoxycaprylylsilane treated titanium oxide | 10 |
| 8 | Triethoxycaprylylsilane treated fine particulate titanium oxide | 6 |
| 9 | Glyceryl tri2-ethylhexanoate | 18 |
| 10 | Water | Remaining amount |
| 11 | Phenoxyethanol | 0.4 |
| 12 | Xanthan gum | 0.05 |
| 13 | Butylene glycol | 1 |
| 14 | Carboxyvinyl polymer | 0.3 |

### (Manufacturing Method)

(1) Components 1 to 4 are mixed.
(2) Components 5 to 9 are mixed using three rolls.
(3) While stirring the mixture obtained in (1) above, the mixture obtained in (2) above is gradually added thereto to form a phase A.
(4) Components 10 to 14 are mixed to form a phase B.
(5) The phase A is added to the phase B, and the mixture is stirred to be uniform.

### (O/W sunscreen)

**[Table 7]**

| Component | | Blending amount |
|---|---|---|
| Phase A | | |
| 1 | (A) Carboxylic acid modified silicone | 1.5 |
| 2 | Glycerin | 5 |
| 3 | 50% KOH aqueous solution | 0.5 |
| 4 | Caprylic/capric triglyceride | 2 |
| 5 | Ethylhexyl methoxycinnamate | 7.5 |
| 6 | Diethylaminohydroxybenzoyl hexyl benzoate | 2 |

| Phase B | | |
|---|---|---|
| 7 | Acrylates copolymer (polymer concentration 30 mass%) | 4 |
| 8 | Water | Residual |
| 9 | Triethanolamine | Appropriate amount |
| 10 | Phenoxyethanol | 0.9 |

| Phase C | | |
|---|---|---|
| 11 | (A) Carboxylic acid modified silicone | 1.3 |
| 12 | Zinc oxide | 8 |
| 13 | Butylene glycol | 6.7 |
| 14 | Water | 10.6 |

| Phase D | | |
|---|---|---|
| 15 | (Divinyl dimethicone/dimethicone) copolymer (and) C12-13 pareth -3 (and) C12-13 pareth-23 (polymer concentration: 60 mass%) | 4.2 |

### (Manufacturing Method)

(1) Components 1 to 3 are mixed.
(2) Components 4 to 6 are mixed.
(3) While stirring the mixture obtained in (1) above, a mixture obtained in (2) above is added in a portion (4) to form a phase A.
(5) The component of phase B is mixed to form the phase B.
(6) The components of Phase C are mixed using three rolls to form a C phase.
(7) The phase A and the phase C are added to the phase B and mixed together.
(8) A phase D is added to the mixture of the phases A, B, and C phase, and the mixture is stirred to be uniform.

### (hand cream)

**[Table 8]**

| Component | | Blending amount |
|---|---|---|
| 1 | Macademia nut oil | 5 |
| 2 | Shea butter | 10 |
| 3 | Glycerin | 2 |
| 4 | (A) Carboxylic acid modified silicone | 3 |
| 5 | Amodimethicone | 1 |
| 6 | Polyperfluoromethylisopropyl ether | 1 |
| 7 | Water | Remaining amount |
| 8 | Triethanolamine | 0.85 |
| 9 | Polyvinylpyrrolidone | 0.4 |
| 10 | Silica | 5 |
| 11 | Butylene glycol | 5 |
| 12 | Methylparaben | 0.25 |
| 13 | Carboxyvinyl polymer | 0.3 |
| 14 | Phenoxyethanol | 0.3 |

### (Manufacturing Method)

(1) Components 1 to 6 are mixed and heated to 80°C to prepare an oil phase (a).
(2) Components 7 to 13 are mixed and heated to 80°C to prepare an aqueous phase (b).
(3) The aqueous phase (b) is mixed little by little with the oil phase (a) to prepare an emulsified phase (c).
(4) The emulsified phase (c) is cooled and mixed with a component 14 at 35°C to prepare a cosmetic composition.

### (Eye shadow)

**[Table 9]**

| Component | | Blending amount |
|---|---|---|
| 1 | Water | 0.75 |
| 2 | Potassium hydroxide | 0.9 |
| 3 | Glycerin | 10 |
| 4 | (A) Carboxylic acid modified silicone | 4 |
| 5 | Glyceryl tri2-ethylhexanoate | 7 |
| 6 | Diisostearyl malate | 2 |
| 7 | Triethoxycaprylylsilane treated titanium oxide | 2.6 |
| 8 | Triethoxycaprylylsilane treated iron oxide | 2.7 |
| 9 | Cyclopentasiloxane (and) (acrylates/polytrimethylsiloxy methacrylate) copolymer (polymer concentration 30 mass%) | 3 |
| 10 | Water | Remaining amount |
| 11 | Butylene glycol | 8 |
| 12 | Pentylene glycol | 2 |
| 13 | (Acrylates/alkyl acrylate (C10-30)) crosspolymer | 0.3 |
| 14 | Synthetic phlogopite | 10 |
| 15 | Mica titanium | 5 |

### (Manufacturing Method)

(1) Components 1 to 4 are mixed.
(2) Components 5 to 9 are mixed using three rolls.
(3) While stirring the mixture obtained in (1) above, the mixture obtained in (2) above is gradually added thereto to form a phase A.
(4) Components 10 to 15 are mixed to form a phase B.
(5) The phase A is added to the phase B, and the mixture is stirred to be uniform.

### (Lip Cream)

**[Table 10]**

| Component | | Blending amount |
|---|---|---|
| 1 | Water | 0.75 |
| 2 | Potassium hydroxide | 0.9 |
| 3 | Glycerin | 5 |
| 4 | Diglycerin | 5 |
| 5 | (A) Carboxylic acid modified silicone | 4 |
| 6 | Diisostearyl malate | 2 |
| 7 | Polybutene | 10 |
| 8 | Dipentaerythrityl Hexahydroxystearate/Hexastearate/Hexarosinate | 2 |
| 9 | Dimer Dilinoleyl Dimer Dilinoleate | 7.5 |
| 10 | Triethoxycaprylylsilane treated titanium oxide | 1 |
| 11 | Triethoxycaprylylsilane treated red No. 202 | 3.12 |
| 12 | Triethoxycaprylylsilane treated red No. 201 | 1.8 |
| 13 | Triethoxycaprylylsilane-treated yellow No. 4 | 0.09 |
| 14 | Cyclopentasiloxane (and) (acrylates/polytrimethylsiloxy methacrylate) copolymer (polymer concentration 30 mass%) | 3 |
| 15 | Water | Remaining amount |
| 16 | Butylene glycol | 8 |
| 17 | Pentylene glycol | 2 |
| 18 | (Acrylates/alkyl acrylate (C10-30)) crosspolymer | 0.3 |

### (Manufacturing Method)

(1) Components 1 to 5 are mixed.
(2) Components 6 to 14 are mixed using three rolls.
(3) While stirring the mixture obtained in (1) above, the mixture obtained in (2) above is gradually added thereto to form a phase A.
(4) Components 15 to 18 are mixed to form a phase B.
(5) The phase A is added to the phase B, and the mixture is stirred to be uniform.

### (O/W emulsion formulations)

**[Table 11]**

| Component | | Blending amount |
|---|---|---|
| Phase A | | |
| 1 | Carboxylic acid modified silicone | 1 |
| 2 | Polysorbate 80 * | 1.2 |
| 3 | Sorbitan sesquistearate | 0.5 |
| 4 | Glyceryl stearate | 1.5 |
| 5 | Behenyl alcohol | 2.5 |
| 6 | Dimethicone (5cs) | 5 |
| 7 | Squalane | 5 |
| 8 | Isotridecyl isononanoate | 5 |
| 9 | Caprylic/Capric Triglyceride | 5 |

| Phase B | | |
|---|---|---|
| 10 | Water | 40.3 |
| 11 | Butylene glycol | 8 |
| 12 | 1% NaOH aqueous solution | 15 |
| 13 | 2% carbomer aqueous solution ** | 10 |

| | | |
|---|---|---|
| * Tween80 (manufactured by Croda K.K.) ** Carbopole 980 (manufactured by Lubrizol) | | |

### (Manufacturing Method)

(1) Components 1 to 9 are mixed and warmed to 75°C (phase A).
(2) The components 10 to 13 are mixed and warmed to 75°C (phase B).
(3) The phase A is added to the phase B while stirring to be emulsified.
(4) The mixture is cooled to 35°C or lower while stirring.

## Claims

1. An oil-in-water emulsion cosmetic composition comprising:
a carboxylic acid modified silicone (A) that is liquid at room temperature (25°C) expressed by a structural formula (1): 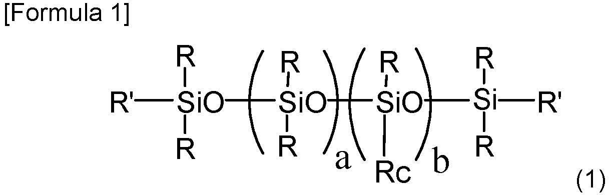 wherein:
Rc represents a carboxyl group-containing organic group represented as a general formula: -R¹-(OR²)p-(O)w-R³-COOH, R1 represents a linear or branched alkylene group having 2 to 22 carbon atoms, R² is a linear or branched alkylene group having 2 to 4 carbon atoms, R³ represents a bond (-) or a linear or branched alkylene group having 1 to 22 carbon atoms, p represents a number of 0, and w represents a number of 0,
R represents a same or different alkyl or alkoxy group, having 1 to 22 carbon atoms, or phenyl group,
R' is Rc or R,
a and b each are a positive number, a + b is a number in a range of 2 to 20, and a/b is in a range of 0.3 to 3.0;
an oil agent (B); and
water (C).

2. The cosmetic composition according to claim 1, wherein a/b in the structural formula (1) is from 0.3 to 2.0.

3. The cosmetic composition according to claim 1 or 2, comprising the carboxylic acid modified silicone (A) in a range of 0.1 to 15 mass% of the total mass of the cosmetic composition.

4. The cosmetic composition according to any one of claims 1 to 3, wherein the oil agent (B) contains at least one ultraviolet light absorbing agent.

5. The cosmetic composition according to any one of claims 1 to 4, comprising the oil agent (B) in a range of 1 to 60 mass% of the total mass of the cosmetic composition.

6. The cosmetic composition according to any one of claims 1 to 5, comprising the water (C) in a range of 20 to 98 mass% of the total mass of the cosmetic composition.

7. The cosmetic composition according to any one of claims 1 to 6, further comprising a basic compound (D).

8. The cosmetic composition according to claim 7, comprising the basic compound (D) in a range of 0.01 to 2.5 mass% of the total mass of the cosmetic composition.

9. The cosmetic composition according to any one of claims 1 to 8, further comprising a polyhydric alcohol (E).

10. The cosmetic composition according to any one of claims 1 to 9, further comprising one or more (F) selected from a film-forming agent (F1) and a water-soluble thickening agent (F2).

11. The cosmetic composition according to any one of claims 1 to 10, further comprising a hydrophobic powder (G).

12. The cosmetic composition according to claim 11, wherein the hydrophobic powder (G) is dispersed in an oil phase and/or an aqueous phase.

13. The cosmetic composition according to any one of claims 1 to 12, comprising less than 5 mass% of a surfactant other than the carboxylic acid modified silicone (A) in the total mass of the cosmetic composition.

## Patentansprüche

1. Öl-in-Wasser-Emulsion-Kosmetikzusammensetzung, umfassend:
ein carbonsäuremodifiziertes Silikon (A), das bei Raumtemperatur (25 °C) flüssig ist, ausgedrückt durch eine Strukturformel (1): 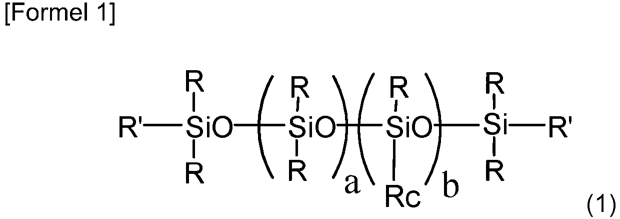 worin:
Rc eine carboxylgruppenenthaltende organische Gruppe darstellt, die als eine allgemeine Formel dargestellt wird: -R¹-(OR²)p-(O)wR³-COOH, R¹ eine lineare oder verzweigte Alkylengruppe, die 2 bis 22 Kohlenstoffatome aufweist, darstellt, R² eine lineare oder verzweigte Alkylengruppe, die 2 bis 4 Kohlenstoffatome aufweist, ist, R³ eine Bindung (-) oder eine lineare oder verzweigte Alkylengruppe, die 1 bis 22 Kohlenstoffatome aufweist, darstellt, p eine Zahl von 0 darstellt und w eine Zahl von 0 darstellt,
R eine gleiche oder unterschiedliche Alkyl- oder Alkoxygruppe, die 1 bis 22 Kohlenstoffatome aufweist, oder eine Phenylgruppe darstellt,
R' Rc oder R ist,
a und b jeweils eine positive Zahl sind, a + b eine Zahl in einem Bereich von 2 bis 20 ist, und a/b in einem Bereich von 0,3 bis 3,0 ist;
ein Ölmittel (B); und
Wasser (C).

2. Kosmetikzusammensetzung nach Anspruch 1, wobei a/b in der Strukturformel (1) von 0,3 bis 2,0 beträgt.

3. Kosmetikzusammensetzung nach Anspruch 1 oder 2, umfassend das carbonsäuremodifizierte Silikon (A) in einem Bereich von 0,1 bis 15 Masse-% bezogen auf die Gesamtmasse der Kosmetikzusammensetzung.

4. Kosmetikzusammensetzung nach einem der Ansprüche 1 bis 3, wobei das Ölmittel (B) mindestens ein ultraviolettlichtabsorbierendes Mittel enthält.

5. Kosmetikzusammensetzung nach einem der Ansprüche 1 bis 4, umfassend das Ölmittel (B) in einem Bereich von 1 bis 60 Masse-% bezogen auf die Gesamtmasse der Kosmetikzusammensetzung.

6. Kosmetikzusammensetzung nach einem der Ansprüche 1 bis 5, umfassend das Wasser (C) in einem Bereich von 20 bis 98 Masse-% bezogen auf die Gesamtmasse der Kosmetikzusammensetzung.

7. Kosmetikzusammensetzung nach einem der Ansprüche 1 bis 6, ferner umfassend eine basische Verbindung (D).

8. Kosmetikzusammensetzung nach Anspruch 7, umfassend die basische Verbindung (D) in einem Bereich von 0,01 bis 2,5 Masse-% bezogen auf die Gesamtmasse der Kosmetikzusammensetzung.

9. Zusammensetzung nach einem der Ansprüche 1 bis 8, ferner umfassend einen Polyalkohol (E).

10. Kosmetikzusammensetzung nach einem der Ansprüche 1 bis 9, ferner umfassend ein oder mehrere (F), ausgewählt aus einem filmbildenden Mittel (F1) und einem wasserlöslichen Verdickungsmittel (F2).

11. Kosmetikzusammensetzung nach einem der Ansprüche 1 bis 10, ferner umfassend ein hydrophobes Pulver (G).

12. Kosmetikzusammensetzung nach Anspruch 11, wobei das hydrophobe Pulver (G) in einer Ölphase und/oder einer wässrigen Phase dispergiert ist.

13. Kosmetikzusammensetzung nach einem der Ansprüche 1 bis 12, umfassend zu weniger als 5 Masse-% ein Tensid anders als das carbonsäuremodifizierte Silikon (A) in der Gesamtmasse der Kosmetikzusammensetzung.

## Revendications

1. Composition cosmétique sous forme d'émulsion huile-dans-eau, comprenant :
une silicone modifiée par un acide carboxylique (A) qui est liquide à température ambiante (25 °C), exprimée par une formule structurelle (1) : 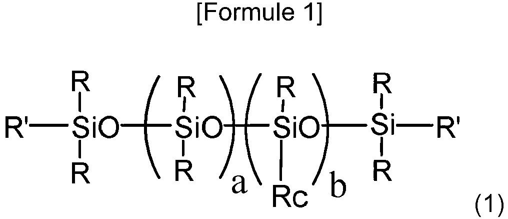 où :
Rc représente un groupe organique contenant un groupe carboxyle représenté sous la forme d'une formule générale : -R¹-(OR²)p-(O)w-R³-COOH, R¹ représente un groupe alkylène linéaire ou ramifié ayant 2 à 22 atomes de carbone, R² est un groupe alkylène linéaire ou ramifié ayant 2 à 4 atomes de carbone, R³ représente une liaison (-) ou un groupe alkylène linéaire ou ramifié ayant 1 à 22 atomes de carbone, p représente un nombre 0, et w représente un nombre 0,
R représente un groupe alkyle ou alcoxy, identique ou différent, ayant de 1 à 22 atomes de carbone, ou un groupe phényle,
R' est Rc ou R,
a et b sont chacun un nombre positif, a + b est un nombre dans une plage de 2 à 20, et a/b est dans une plage de 0,3 à 3,0 ;
un agent huileux (B) ; et
de l'eau (C).

2. Composition cosmétique selon la revendication 1, dans laquelle alb dans la formule structurelle (1) est de 0,3 à 2,0.

3. Composition cosmétique selon la revendication 1 ou 2, comprenant la silicone modifiée par un acide carboxylique (A) dans une plage de 0,1 à 15 % en masse de la masse totale de la composition cosmétique.

4. Composition cosmétique selon l'une quelconque des revendications 1 à 3, dans laquelle l'agent huileux (B) contient au moins un agent absorbant la lumière ultraviolette.

5. Composition cosmétique selon l'une quelconque des revendications 1 à 4, comprenant l'agent huileux (B) dans une plage de 1 à 60 % en masse de la masse totale de la composition cosmétique.

6. Composition cosmétique selon l'une quelconque des revendications 1 à 5, comprenant l'eau (C) dans une plage de 20 à 98 % en masse de la masse totale de la composition cosmétique.

7. Composition cosmétique selon l'une quelconque des revendications 1 à 6, comprenant en outre un composé basique (D).

8. Composition cosmétique selon la revendication 7, comprenant le composé basique (D) dans une plage de 0,01 à 2,5 % en masse de la masse totale de la composition cosmétique.

9. Composition cosmétique selon l'une quelconque des revendications 1 à 8, comprenant en outre un alcool polyhydrique (E).

10. Composition cosmétique selon l'une quelconque des revendications 1 à 9, comprenant en outre un ou plusieurs (F) choisis parmi un agent filmogène (F1) et un agent épaississant hydrosoluble (F2).

11. Composition cosmétique selon l'une quelconque des revendications 1 à 10, comprenant en outre une poudre hydrophobe (G).

12. Composition cosmétique selon la revendication 11, dans laquelle la poudre hydrophobe (G) est dispersée dans une phase huileuse et/ou une phase aqueuse.

13. Composition cosmétique selon l'une quelconque des revendications 1 à 12, comprenant moins de 5 % en masse d'un tensioactif autre que la silicone modifiée par un acide carboxylique (A) dans la masse totale de la composition cosmétique.
